(19) Europäisches Patentamt
European Patent Office
Office européen des brevets

(11) Veröffentlichungsnummer: **0 556 671 A2**

(12) **EUROPÄISCHE PATENTANMELDUNG**

(21) Anmeldenummer: **93101836.0**

(22) Anmeldetag: **05.02.93**

(51) Int. Cl.[5]: **C07D 471/04**, A01N 43/90, B27K 3/34, //(C07D471/04, 249:00,221:00)

(30) Priorität: **18.02.92 DE 4204816**

(43) Veröffentlichungstag der Anmeldung: **25.08.93 Patentblatt 93/34**

(84) Benannte Vertragsstaaten: **BE CH DE DK ES FR GB IT LI NL**

(71) Anmelder: **BAYER AG**
**D-51368 Leverkusen(DE)**

(72) Erfinder: **Jautelat, Manfred, Dr.**
**Müllersbaum 28**
**W-5093 Burscheid(DE)**
Erfinder: **Dutzmann, Stefan, Dr.**
**Kosenberg 10**
**W-4010 Hilden(DE)**
Erfinder: **Dehne, Heinz-Wilhelm, Dr. habil.**
**Krischer Strasse 81**
**W-4019 Monheim(DE)**

(54) **Triazolo-pyridin-Derivate, Verfahren zu deren Herstellung und deren Verwendung als Mikrobizide.**

(57) Neue Triazolo-pyridin-Derivate der Formel

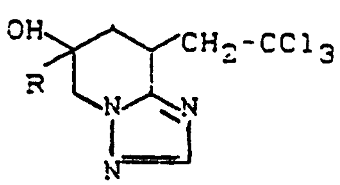

(I)

in welcher

R für gegebenenfalls substituiertes Alkyl, gegebenenfalls substituiertes Cycloalkyl oder gegebenenfalls substituiertes Aryl steht,

sowie deren Säureadditions-Salze und Metallsalz-Komplexe,
ein Verfahren zur Herstellung der neuen Stoffe und deren Verwendung als Mikrobizide im Pflanzenschutz und im Materialschutz.

EP 0 556 671 A2

Die vorliegende Erfindung betrifft neue Triazolo-pyridin-Derivate, ein Verfahren zu deren Herstellung und deren Verwendung als Mikrobizide im Pflanzenschutz und im Materialschutz.

Es ist bereits bekannt geworden, daß bestimmte Dihalogenalkyl-triazolyl-Derivatefungizide Eigenschaften besitzen (vergl. EP-OS 0 097 425). So lassen sich zum Beispiel 4-(2,4-Dichlor-phenyl)-1,2-dibrom-4-hydroxy-5-(1,2,4-triazol-1-yl)-pent-1-en und 4-(2,4-Dichlor-phenyl)-1,2-dichlor-4-hydroxy-5-(1,2,4-triazol-1-yl)-pent-1-en zur Bekämpfung von Pilzen einsetzen. Die Wirkung dieser Stoffe ist gut, läßt aber bei niedrigen Aufwandmengen zu wünschen übrig.

Es wurden nun neue Triazolo-pyridin-Derivate der Formel

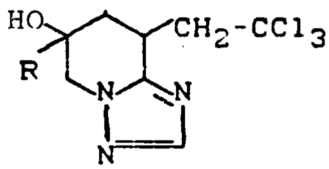

(I)

in welcher

R für gegebenenfalls substituiertes Alkyl, gegebenenfalls substituiertes Cycloalkyl oder gegebenenfalls substituiertes Aryl steht,

sowie deren Säureadditions-Salze und Metallsalz-Komplexe gefunden.

Die Verbindungen der Formel (I) enthalten mindestens ein asymmetrisch substituiertes Kohlenstoffatom und können deshalb in optischen Isomerenformen anfallen. Die vorliegende Erfindung betrifft sowohl die Isomerengemische als auch die einzelnen Isomeren.

Weiterhin wurde gefunden, daß man Triazolo-pyridin-Derivate der Formel (I) sowie deren Säureadditions-Salze und Metallsalz-Komplexe erhält, wenn man Triazolyl-Derivate der Formel

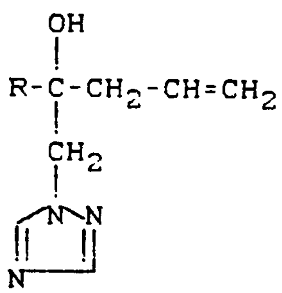

(II)

in welcher

R die oben angegebene Bedeutung hat,

mit Tetrachlorkohlenstoff in Gegenwart eines Verdünnungsmittels unter radikalischen Bedingungen umsetzt und gegebenenfalls anschließend an die so erhaltenen Verbindungen der Formel (I) eine Säure oder ein Metallsalz addiert.

Schließlich wurde gefunden, daß die neuen Triazolo-pyridin-Derivate der Formel (I) sowie deren Säureadditions-Salze und Metallsalz-Komplexe sehr gute mikrobizide Eigenschaften besitzen und sowohl im Pflanzenschutz als auch im Materialschutz eingesetzt werden können.

Überraschenderweise zeigen die erfindungsgemäßen Stoffe wesentlich bessere fungizide Wirksamkeit als die konstitutionell ähnlichsten vorbekannten Verbindungen gleicher Wirkungsrichtung.

Die erfindungsgemäßen Triazolo-pyridin-Derivate sind durch die Formel (I) allgemein definiert.

R steht vorzugsweise für geradkettiges oder verzweigtes Alkyl mit 1 bis 6 Kohlenstoffatomen, wobei jeder dieser Reste einfach bis dreifach, gleichartig oder verschieden substituiert sein kann durch Halogen, Cycloalkyl mit 3 bis 7 Kohlenstoffatomen, Phenyl und/oder Halogenphenyl, oder

für Cycloalkyl mit 3 bis 7 Kohlenstoffatomen, wobei jeder dieser Cycloalkylreste einfach bis dreifach, gleichartig oder verschieden substituiert sein kann durch Halogen und/oder Alkyl mit 1 bis 4 Kohlenstoffatomen, oder

für Phenyl, das einfach bis dreifach, gleichartig oder verschieden substituiert sein kann durch

Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano.

Besonders bevorzugt steht

R für Methyl, Ethyl, n-Propyl, Isopropyl, n-Butyl, tert.-Butyl, tert.-Pentyl, 1-Ethyl-1-methyl-propyl, 1,1-Dimethyl-propyl oder 1,1,2-Trimethylpropyl, wobei jeder dieser zuvorgenannten Reste einfach bis dreifach, gleichartig oder verschieden substituiert sein kann durch Fluor, Chlor, Brom, Phenyl, Chlorphenyl, Dichlorphenyl, Fluorphenyl und/oder Difluorphenyl, oder

für 1-Methyl-cyclohexyl, Cyclohexyl, 1-Fluor-cyclopropyl, 1-Chlor-cyclopropyl, 1-Methylcyclopropyl, Cyclopropyl, 1-Methylcyclopentyl, Cyclopentyl oder 1-Ethyl-cyclopentyl, oder

für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano.

Bevorzugte erfindungsgemäße Stoffe sind auch Additionsprodukte aus Säuren und Triazolo-pyridin-Derivaten der Formel (I), in denen R die Bedeutungen hat, die oben als bevorzugt oder besonders bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, sowie Saccharin und Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und Triazolopyridin-Derivaten der Formel (I), in denen R die Bedeutungen hat, die oben als bevorzugt oder besonders bevorzugt genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in dein folgenden Tabelle aufgeführten Triazolopyridin-Derivate genannt.

Tabelle 1

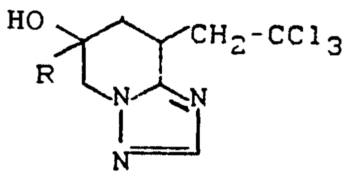

(I)

| R | R |
|---|---|
| | $-C_3H_7$-iso |
| $-CH_3$ | $-C_4H_9$-n |
| $-C_2H_5$ | $-C_4H_9$-iso |
| $-C_3H_7$-n | $-C_4H_9$-sek. |

Tabelle 1 (Fortsetzung)

| R | R |
|---|---|
| 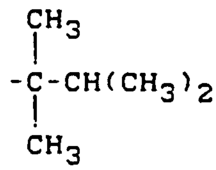 | 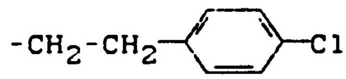 |
| 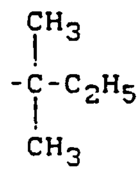 | 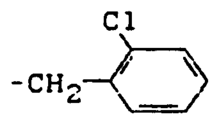 |
| 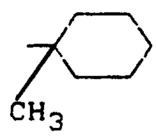 | 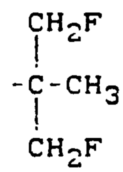 |
| 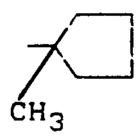 | 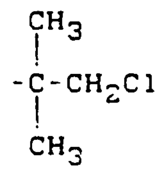 |
| 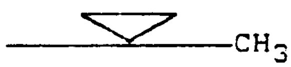 | 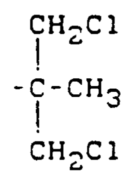 |
| 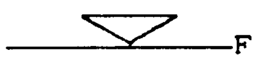 | 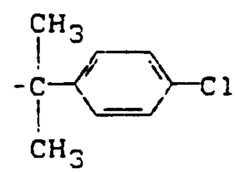 |
| 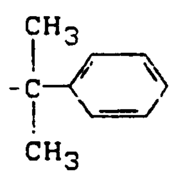 | 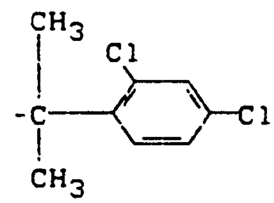 |
| 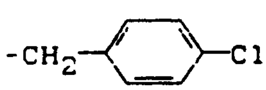 | 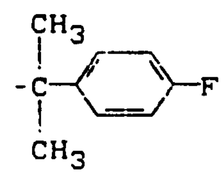 |
| 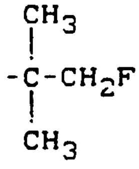 | |

Tabelle 1 (Fortsetzung)

| R | R |
|---|---|
| 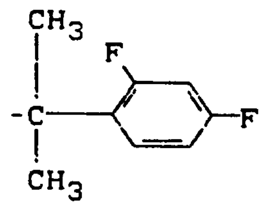 | 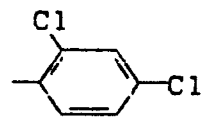 |
| 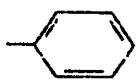 | 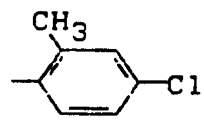 |
| 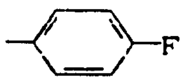 | 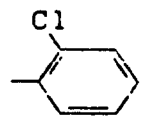 |
| 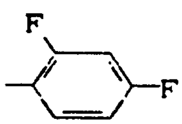 | 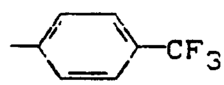 |
| 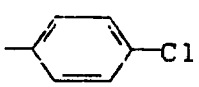 | 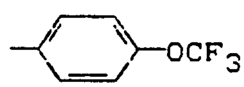 |
| | 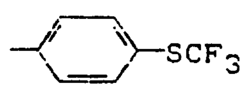 |

Verwendet man 4-(1-Chlor-cyclopropyl)-4-hydroxy-5-(1,2,4-triazol-1-yl)-pent-1-en als Ausgangsstoff, Tetrachlorkohlenstoff als Reaktionskomponente und Dibenzoylperoxid als Radikalbildner, so kann der Verlauf des erfindungsgemäßen Verfahrens durch das folgende Formelschema veranschaulicht werden:

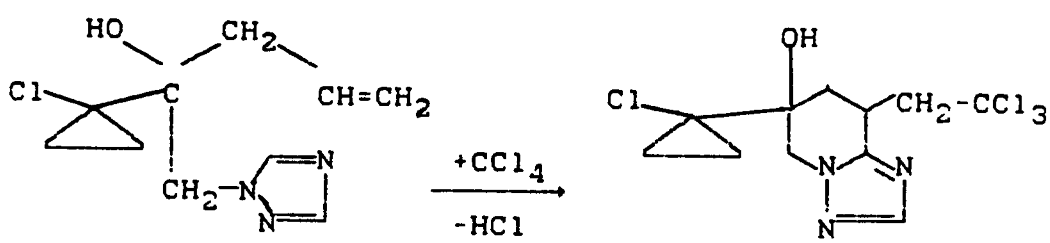


Die bei der Durchführung des erfindungsgemäßen Verfahrens als Ausgangsstoffe benötigten Triazolyl-Derivate der Formel (II) sind teilweise bekannt (vergl. EP-OS 0 097 425). Sie lassen sich herstellen, indem man

a) Triazolylmethyl-ketone der Formel

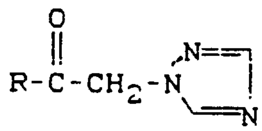

(III)

in welcher

R die oben angegebene Bedeutung hat,

mit Allylhalogeniden der Formel

$CH_2 = CH\text{-}CH_2\text{-}Hal$ (IV)

in welcher

Hal für Chlor oder Brom steht,

in Gegenwart von aktiviertem Zink, Magnesium oder Aluminium und in Gegenwart eines Verdünnungsmittels umsetzt,

oder

b) Chlormethylketone der Formel

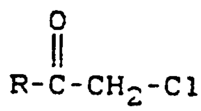

(V)

in welcher

R die oben angegebene Bedeutung hat,

mit Allylhalogeniden der Formel

$CH_2 = CH\text{-}CH_2\text{-}Hal$ (IV)

in welcher

Hal die oben angegebene Bedeutung hat,

in Gegenwart von aktiviertem Zink, Magnesium oder aktiviertem Aluminium und in Gegenwart eines Verdünnungsmittels umsetzt und dann die dabei entstehenden Hydroxyalkene der Formel

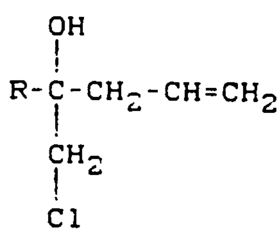

(VI)

in welcher

R die oben angegebene Bedeutung hat,

mit 1,2,4-Triazol der Formel

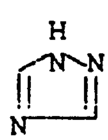

(VII)

in Gegenwart eines Säurebindemittels und in Gegenwart eines Verdünnungsmittels umsetzt.

Die bei der Durchführung des Verfahrens (a) als Ausgangsstoffe benötigten Triazolyl-methyl-ketone der Formel (III) sind bekannt oder lassen sich nach pinzipiell bekannten Verfahren in einfacher Weise herstellen (vergl. DE-OS 2 431 407 und EP-OS 0 353 558).

Die bei der Durchführung des Verfahrens (a) als Reaktionskomponenten benötigten Allylhalogenide der Formel (IV) sind bekannt.

Als Metalle kommen bei der Durchführung des Verfahrens (a) aktiviertes Zink, Magnesium oder aktiviertes Aluminium in Betracht. Die Metalle werden dabei in Form von Pulver, Spänen oder Schuppen unter Zugabe von Iod als Aktivator eingesetzt. Aktiviertes Aluminium wird hergestellt, indem man zu Aluminium-Schuppen katalytische Mengen an Quecksilber-(II)-chlorid und Iod zugibt.

Als Verdünnungsmittel können bei der Durchführung des Verfahrens (a) alle für derartige Umsetzungen üblichen inerten, organischen Solventien verwendet werden. Vorzugsweise in Frage kommen Ether, wie Tetrahydrofuran, Diethylether, Dioxan, Dimethoxyethan und Diethylenglykol-di-methylether (Diglyme), und außerdem auch aromatische Kohlenwasserstoffe, wie Toluol, gegebenenfalls im Gemisch mit einem Ether.

Die Reaktionstemperaturen können bei der Durchführung des Verfahrens (a) in einem bestimmten Bereich variiert werden. Im allgemeinen arbeitet man bei Temperaturen zwischen -20°C und +80°C, voruzugsweise zwischen 0°C und +60°C.

Bei der Durchführung des Verfahrens (a) arbeitet man im allgemeinen unter Normaldruck. Es ist aber auch möglich, unter erhöhtem oder vermindetem Druck zu arbeiten.

Bei der Durchführung des Verfahrens (a) geht man so vor, daß man auf 1 Mol an Triazolylmethyl-keton der Formel (III) eine äquimolare Menge oder auch einen Überschuß an Allylhalogenid der Formel (IV) sowie ebenfalls eine äquimolare Menge oder auch einen Überschuß an aktiviertem Zink, Magnesium beziehungsweise an Aluminium zusammen mit einer katalytischen Menge an Quecksilber(II)-chlorid und Iod einsetzt. Die Isolierung der entstehenden Produkte erfolgt nach üblichen Methoden. Im allgemeinen geht man so vor, daß man das Reaktionsgemisch einengt, mit gesättigter, wäßriger Ammoniumchlorid-Lösung und einem mit Wasser wenig mischbaren organischen Lösungsmittel versetzt, die organische Phase abtrennt und nach dem Trocknen einengt.

Die bei der Durchführung des Verfahrens (b) als Ausgangsstoffe benötigten Chlormethylketone der Formel (V) sind bekannt oder lassen sich nach prinzipiell bekannten Methoden herstellen (vergl. DE-OS 3 049 461).

Die Durchführung der ersten Stufe des Verfahrens (b) erfolgt unter den Bedingungen, die auch bei der Durchführung des Verfahrens (a) angewandt werden.

Die Hydroxyalkene der Formel (VI) können direkt weiter umgesetzt werden mit 1,2,4-Triazol der Formel (VII). Sie können aber auch zunächst in Oxirane überführt werden und dann mit 1,2,4-Triazol der Formel (VII) umgesetzt werden.

Bei der Durchführung der zweiten Stufe des Verfahrens (b) kommen als Säurebindemittel alle üblichen Säureakzeptoren in Frage. Vorzugsweise verwendbar sind Alkalimetallcarbonate und -hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzyl-amin und Pyridin, und außerdem cyclische Amine, wie 1,5-Diaza-bicyclo-[4,3,0]non-5-en (DBN), 1,8 Diaza-bicyclo[5,4,0]-undec-7-en (DBU) und 1,4-Diaza-bicyclo[2,2,2]octan (DABCO).

Als Verdünnungsmittel kommen bei der Durchführung der zweiten Stufe des Verfahrens (b) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform: Tetrachlorkohlenstoff, Chlorbenzol und o-Dichlorbenzol, Ether wie Diethyl- und Dibutylether, sowie tert.-Butylmethylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (b) innerhalb eines größeren Bereiches variiert werden. Im allgemeinen arbeitet man bei Temperaturen zwischen 0°C und 200°C, vorzugsweise zwischen 20°C und 150°C.

Bei der Durchführung der zweiten Stufe des Verfahrens (b) geht man im allgemeinen so vor, daß man auf 1 Mol an Hydroxyalken der Formel (VI) eine äquivalente Menge oder auch einen Überschuß an 1,2,4-Triazol der Formel (VII) sowie 2 bis 3 Mol an Säurebindemittel einsetzt. Die Aufarbeitung erfolgt nach üblichen Methoden.

Als Radikalbildner kommen bei der Durchführung des erfindungsgemäßen Verfahrens alle für derartige Zwecke üblicherweise verwendbaren Substanzen in Betracht. Vorzugsweise in Frage kommen Peroxide, wie Dibenzoylperoxid, ferner Hydroperoxide, wie tert.-Butyl-hydroperoxid, und außerdem Azo-bis-isobutyronitril.

Zweckmäßigerweise arbeitet man unter Bestrahlung mit UV-Licht.

Als Verdünnungsmittel kommen bei der Durchführung des erfindungsgemäßen Verfahrens alle üblichen inerten organischen Solventien in Betracht. Vorzugsweise verwendbar sind cycloaliphatische oder aromatische, gegebenenfalls chlorierte Kohlenwasserstoffe, wie Cyclohexan, Benzol oder Chlorbenzol, und weiterhin auch chlorierte aliphatische Kohlenwasserstoffe, wie 1,2-Dichlorethan oder Chloroform.

Die Reaktionstemperaturen können bei der Durchführung des erfindungsgemäßen Verfahrens innerhalb eines größeren Bereiches variiert werden. Im allgemeinen arbeitet man bei Temperaturen zwischen 20°C und 140°C, vorzugsweise zwischen 40°C und 120°C.

Das erfindungsgemäße Verfahren wird im allgemeinen unter Normaldruck durchgeführt. Es ist aber auch möglich, unter erhöhtem Druck zu arbeiten.

Bei der Durchführung des erfindungsgemäßen Verfahrens setzt man auf 1 Mol an Triazolyl-Derivat der Formel (II) mindestens eine äquimolare Menge, vorzugsweise aber einen Überschuß an Tetrachlorkohlenstoff und eine katalytische Menge an Radikalbildner ein. Die Aufarbeitung erfolgt nach üblichen Methoden. Im allgemeinen geht man so vor, daß man das Reaktionsgemisch einengt, den verbleibenden Rückstand mit wäßriger Alkalimetallcarbonat-Lösung sowie mit einem mit Wasser wenig mischbaren organischen Lösungsmittel versetzt, die organische Phase abtrennt und nach dem Trocknen einengt. Die anfallenden Produkte können gegebenenfalls nach üblichen Methoden weiter gereinigt werden.

Die erfindungsgemäßen Triazolo-pyridin-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden,

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;

Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Cochliobolus sativus,Pyrenophora teres, Pseudocercosporella herpotrichoides, sowie Erysiphe und Fusarium-Arten. Außerdem zeigen die erfindungsgemäßen Stoffe eine sehr gute Wirkung gegen Venturia, Sphaerotheca und Botrytis. Sie besitzen ferner eine gute in-vitro-Wirkung und zeigen auch akarizide Eigenschaften.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophilla,
Trichoderma, wie Trichoderma viride,
Escherichia, wie Escherichia coli,
Pseudomonas, wie Pseudomonas aeruginosa,
Staphylococcus, wie Staphylococcus aureus.

Die erfindungsgemäßen Stoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als

Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei Verwendung im Pflanzenschutz als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je kilogramm Saatgut, vorzugsweise 0,01 bis 10 g benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt:

Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, Trihalogenmethylthio-Verbindungen wie Folpet, Fluorfolpet, Dichlorfluanid.

Die Herstellung und die Verwendung der erfindungsgemäßen Wirkstoffe werden durch die folgenden Beispiele veranschaulicht.

## Herstellungsbeispiele

### Beispiel 1

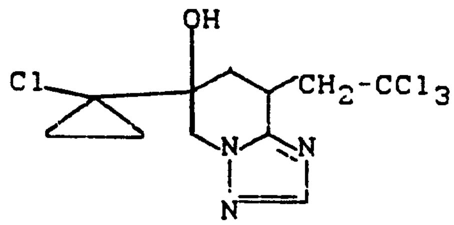

(I-1)

Ein Gemisch aus 22,7 g (0,1 Mol) 4-(1-Chlor-cyclopropyl)-4-hydroxy-5-(1,2,4-triazol-1-yl)-pent-1-en, 200 ml Cyclohexan, 100 ml Tetrachlorkohlenstoff und 1 g Dibenzoylperoxid wird unter Bestrahlung mit UV-Licht 150 Stunden unter Rückfluß erhitzt. Danach werden das Lösungsmittel und überschüssiger Tetrachlorkohlenstoff bei Normaldruck abdestilliert. Der verbleibende Rückstand wird mit wäßriger Natriumcarbonat-Lösung und Dichlormethan versetzt. Die organische Phase wird abgetrennt und nach dem Trocknen über Natriumsulfat unter vermindertem Druck eingeengt. Das dabei erhaltene Rohprodukt wird mit einem Gemisch aus Hexan/Essigester = 1 : 1 als Laufmittel an Kieselgel chromatographiert. Nach dem Einengen des Eluates erhält man 9,6 g (28 % der Theorie) an 7-(1-Chlor-cyclopropyl)-7-hydroxy-5-(2,2,2-trichlorethyl)-5,6,7,8-tetrahydro-1,2,4-triazolo[2,3-a]-pyridin in Form einer Festsubstanz vom Schmelzpunkt 153 bis 154 °C.

### Herstellung von Ausgangssubstanzen

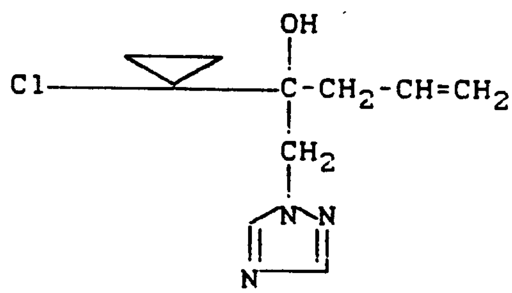

(II-1)

In ein Gemisch aus 7,2 g (0,11 Mol) Zinkstaub, 0,1 g Iod und 50 ml absolutem Tetrahydrofuran wird bei 20 °C unter Stickstoffatmosphäre und unter Rühren innerhalb von 0,5 Stunden eine Lösung von 12,1 g (0,1 Mol) Allylbromid und 16,65 g (0,09 Mol) 1-(1-Chlorcyclopropyl)-2-(1,2,4-triazol-1-yl)-ethan-1-on in 50 ml Tetrahydrofuran eingetropft. Nach beendeter Zugabe rührt man noch 24 Stunden bei Raumtemperatur, engt dann durch Abziehen des Lösungsmittels ein und versetzt mit wäßriger Ammoniumchlorid-Lösung. Das entstehende Gemisch wird mehrfach mit Dichlormethan extrahiert. Die vereinigten organischen Phasen werden nach dem Trocknen über Natriumsulfat durch Abziehen des Lösungsmittels unter vermindertem Druck eingeengt. Man erhält auf diese Weise 10,2 g (50 % der Theorie) an 4-(1-Chlor-cyclopropyl)-4-hydroxy-5-(1,2,4-triazol-1-yl)-pent-1-en in Form einer Festsubstanz vom Schmelzpunkt 75 bis 76 °C.

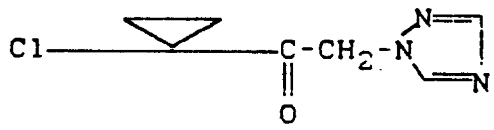

(III-1)

Zu einer unter Rückfluß kochenden Suspension von 83 g (0,6 Mol) Kaliumcarbonat und 58 g (0,84 Mol) Triazol in 330 ml Acetonitril werden 100 g (0,66 Mol) 1-Chlor-cyclopropyl-chlormethyl-keton in 80 ml Acetonitril zugetropft. Es wird 8 Stunden unter Rückfluß gekocht, dann abgesaugt und eingeengt. Der Rückstand wird in Ethylacetat/Toluol aufgenommen, mit Wasser gewaschen, über Natriumsulfat getrocknet und eingeengt. Die anschließende säulenchromatographische Reinigung mit dem Laufmittel Dichlormethan lieferte 62 g (51 % der Theorie) an 1-(1-Chlorcyclopropyl)-2-(1,2,4-triazol-1-yl)-ethan-1-on.

Beispiel 2

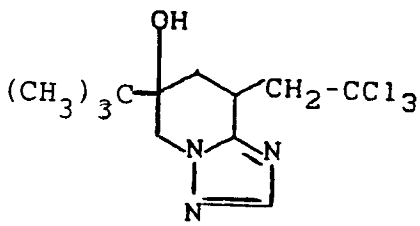

(I-2)

Die Verbindung der Formel (I-2) wurde nach der im Beispiel 1 beschriebenen Methode hergestellt.
Schmelzpunkt: 201 - 203 °C

Verwendungsbeispiele

Beispiel A

Erysiphe-Test (Gerste)/protektiv
Lösungsmittel: 100 Gew.-Teile Dimethylformamid
Emulgator: 0,25 Gew.-Teile Alkylarylpolyglykolether
Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzbrühe einen Wirkungsgrad von 100%.

Beispiel B

Erysiphe-Test (Gerste) / kurativ
Lösungsmittel: 100 Gew.-Teile Dimethylformamid
Emulgator: 0,25 Gew.-Teile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f. sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20°C und einer relativen Luftfeuchtigkein von ca. 80% aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzbrühe einen Wirkungsgrad von 100%.

Beispiel C

Erysiphe-Test (Weizen) / kurativ

Lösungsmittel: 100 Gew.-Teile Dimethylformamid

Emulgator: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f. sp. tritici bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzbrühe einen Wirkungsgrad von 100%.

Beispiel D

Leptosphaeria nodorum-Test (Weizen) / protektiv

Lösungsmittel: 100 Gew.-Teile Dimethylformamid

Emulgator: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigt die erfindungsgemäße Verbindung (I-1) bei einer Konzentration von 250 ppm in der Spritzbrühe einen Wirkungsgrad von 100%.

Beispiel E

Uncinula-Test (Rebe) / protektiv

Lösungsmittel: 4,7 Gew.-Teile Aceton

Emulgator: 0,3 Gew.-Teile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend bei 23 bis 24°C und bei einer relativen Luftfeuchtigkeit von ca. 75 % im Gewächshaus aufgestellt.

14 Tage nach der Inokulation erfolgt die Auswertung.

In diesem Test zeigt die erfindungsgemäße Verbindung (I-1) bei einer Konzentration von 10 ppm in der Spritzbrühe einen Wirkungsgrad von 100%.

Beispiel F

Venturia-Test (Apfel) / kurativ
Lösungsmittel: 4,7 Gew.-Teile Aceton
Emulgator: 0,3 Gew.-Teile Alkylarylpolyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Konidiensuspension des Apfelschorferregers (Venturia inaequalis) inokuliert. Die Pflanzen verbleiben 1 Tag bei 20 °C und 100 % relativer Luftfeuchtigkeit in einer Inkubationskabine und werden dann im Gewächshaus aufgestellt. Nach einer angegebenen Stundenzahl werden die Pflanzen mit der Wirkstoffzubereitung tropfnaß gespritzt.

Die Pflanzen werden in einem Gewächshaus bei einer Temperatur von ca. 20 °C und einer relativen Luftfeuchtigkeit von ca. 70 % aufgestellt.

12 Tage nach der Inokulation erfolgt die Auswertung.

In diesem Test zeigt die erfindungsgemäße Verbindung (I-1) bei einer Konzentration von 20 ppm in der Spritzbrühe einen Wirkungsgrad von 100%.

**Patentansprüche**

**1.** Triazolo-pyridin-Derivate der Formel

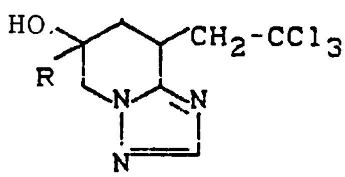

(I)

in welcher

R für gegebenenfalls substituiertes Alkyl, gegebenenfalls subsituiertes Cycloalkyl oder gegebenenfalls substituiertes Aryl steht,

sowie deren Säureadditions-Salze und Metallsalz-Komplexe.

**2.** Triazolo-pyridin-Derivate der Formel (I) gemäß Anspruch 1, in denen

R für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen, Cycloalkyl mit 3 bis 7 Kohlenstoffatomen, Phenyl und/oder Halogenphenyl, oder für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen, oder für Phenyl steht, das einfach bis dreifach, gleichartig oder verschieden substituiert sein kann durch Halogen Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano.

**3.** Verfahren zur Herstellung von Triazolo-pyridin-Derivaten der Formel

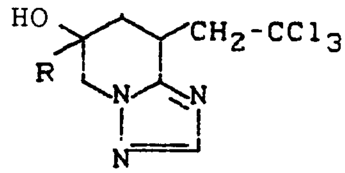

(I)

in welcher

R für gegebenenfalls substituiertes Alkyl, gegebenenfalls substituiertes Cycloalkyl oder gegebenenfalls substituiertes Aryl steht,

sowie von deren Säureadditions-Salzen und Metallsalz-Komplexen, dadurch gekennzeichnet, daß man Triazolyl-Derivate der Formel

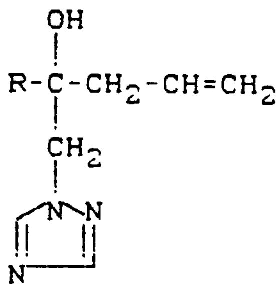

(II)

in welcher

R die oben angegebene Bedeutung hat,

mit Tetrachlorkohlenstoff in Gegenwart eines Verdünnungsmittels unter radikalischen Bedingungen umsetzt,

und gegebenenfalls anschließend an die so erhaltenen Verbindungen der Formel (I) eine Säure oder ein Metallsalz addiert.

4. Mikrobizide Mittel, gekennzeichnet durch einen Gehalt an mindestens einem Triazolo-pyridin-Derivat der Formel (I) gemäß Anspruch 1 bzw. an einem Säureadditions-Salz oder Metallsalz-Komplex eines Triazolo-pyridin-Derivates der Formel (I).

5. Verwendung von Triazolo-pyridin-Derivaten der Formel (I) gemäß Anspruch 1 bzw. von deren Säureadditions-Salzen und Metallsalz-Komplexen als Mikrobizide im Pflanzenschutz und im Materialschutz.

6. Verfahren zur Bekämpfung von unerwünschten Mikroorganismen im Pflanzenschutz und im Materialschutz dadurch gekennzeichnet, daß man Triazolo-pyridin-Derivate der Formel (I) gemäß Anspruch 1 bzw. deren Säureadditions-Salze oder Metallsalz-Komplexe auf die Mikroorganismen und/oder deren Lebensraum ausbringt.

7. Verfahren zur Herstellung von mikrobiziden Mitteln, dadurch gekennzeichnet, daß man Triazolo-pyridin-Derivate der Formel (I) gemäß Anspruch 1 bzw. deren Metallsalz-Komplexe oder Säureadditions-Salze mit Streckmitteln und/oder oberflächenaktiven Stoffen vermischt.

8. Triazolo-pyridin-Derivat gemäß Anspruch 1, gekennzeichnet durch die Formel

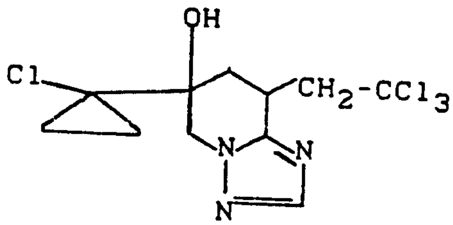

**9.** Triazolo-pyridin-Derivat gemäß Anspruch 1, gekennzeichnet durch die Formel